# EUROPEAN PATENT APPLICATION

(11) **EP 1 779 794 A1**
(43) Date of publication of application: **02.05.2007**
(21) Application number: 06255389.6
(22) Date of filing: 20.10.2006
(51) Int. Cl.: A61B 17/72, A61B 17/78

(54) **Orthopaedic nail with oblique openings**

(30) Priority: 31.10.2005 US 263199
(71) Applicant: DePuy Products, Inc., Warsaw, IN 46581 (US)
(72) Inventor: Czartoski, Timothy J., Fort Wayne, IN 46814 (US); Davidson, Dale G., Akron, OH 44319 (US); Muhammad, William, Fort Wayne, IN 46835 (US); Cole, J. Dean, Orlando, FL 32804 (US); Moed, Berton R., St Louis, MO 63105 (US); Watson, Tracy J., Town & Country, MO 63131 (US); Wich, Michael Karl-Heinz, Berlin (DE)
(74) Representative: Belcher, Simon James

(57) **Abstract**

An intramedullary nail (12,112,12A,12B,212,312,412) for use in a medullary canal (6) of a long bone (4) includes a body defining a longitudinal axis (14,114,214,314,414) and an external periphery of the body for fitting in the medullary canal of the long bone. The body has a first internal wall (18) of the body defining a first opening (20,26,42,62,64,66,68,64B,66B,68B,76B) through the body. The first opening defines a first opening centerline. The body has a second internal wall (24) of the body defining a second opening (20,26,42,62,64,68,64B,66B,68B,76B) through the body. The second opening defines a second opening centerline. The first opening centerline and the second opening centerline are oblique with respect to each other. At least one of the first opening centerline and the second opening centerline are transverse to the longitudinal axis of said body.

## Description

The present invention relates generally to the field of orthopaedics, and more particularly, to a device for securing a prosthetic component to bone for use in with orthopaedic trauma or orthopaedic joint products.

The skeletal system includes many long bones that extend from the human torso. These long bones include the femur, fibula, tibia, humerus, radius and ulna. These long bones are particularly exposed to trauma from accidents, and, as such, often are fractured during a trauma and may be subject to complex devastating fractures.

Automobile accidents, for instance, are a common cause of trauma to long bones. In particular, the femur and tibia frequently fracture when the area around the knee is subjected to a frontal automobile accident.

Often the distal end or proximal portions of the long bone, for example the femur and the tibia, are fractured into several components and must be realigned. Mechanical devices, commonly in the forms of pins, plates, screws, nails, wires and external devices are commonly used to attach fractured long bones. The pins, plates, wires, nails and screws are typically made of a durable material compatible to the human body, for example titanium, stainless steel or cobalt chromium.

Fractures of the long bone are typically secured into position by at least one of three possible techniques or methods.

The first method is the use of intramedullary nails that are positioned in the intramedullary canal of those portions of the fractured bone.

A second method of repairing fractured bones is the use of internal bone plates that are positioned under the soft tissue and on the exterior of the bone which bridge the fractured portion of the bone.

Another method of securing fractured bones in position is the use of external fixators. These external fixators have at least two general categories. In one category, the fixator is generally linear with a first portion of the fixator to connect to a first fracture segment of the bone and a second fracture segment of the fixator to connect to the second fracture segment of the bone. A first series of bone screws or pins are first connected to the fixator and then into the first portion of the bone. Then, a second series of screws or pins are connected to the fixator and then to the second fracture segment of the bone, thereby securing the first fracture segment of the bone to the second fracture segment of the bone.

A second method of external fixation is through the use of a ring type fixator that uses a series of spaced-apart rings to secure the bone. For example, an upper ring and a lower ring are spaced apart by rods. A plurality of wires are placed through the long bone and are connected on each end of the long bone by the ring. The wires are then tensioned much as spokes in a bicycle are tightened, thereby providing for a rigid structure to support the first fracture segment portion of the bone. Similarly, a plurality of wires are positioned through the second fracture segment of the bone and are secured to and tensioned by the lower ring to provide a rigid fixation of the second fracture segment of the bone bridging the fracture site.

There are a variety of devices used to treat femoral fractures. Fractures of the neck, head or inter-trochanter of the femur have been successfully treated with a variety of compression screw assemblies, which include generally a compression plate having a barrel member, a lag screw and a compressing screw. The compression plate is secured to the exterior of the femur and the barrel member is inserted into a predrilled hole in the direction of the femoral head.

The lag screw, which has a threaded end and a smooth portion, is inserted through the barrel member so that it extends across the break and into the femoral head. The threaded portion engages the femoral head. The compressing screw connects the lag screw to the plate. By adjusting the tension of the compressing screw the compression (reduction) of the fracture can be adjusted. The smooth portion of the lag screw must be free to slide through the barrel member to permit the adjustment of the compression screw.

Subtrochanteric and femoral shaft fractures have been treated with the help of intramedullary rods, which are inserted into the marrow canal of the femur to immobilize the femur parts involved in fractures. A single angled cross-nail or locking screw is inserted through the femur and the proximal end of the intramedullary rod. In some varieties, one or two screws may also be inserted through the femoral shaft and through the distal end of the intramedullary rod. The standard intramedullary rods have been successfully employed in treating fractures in lower portions of the femoral shaft.

The proximal femoral fractures, for example, those around the lesser trochanter, greater trochanter, and femoral neck have been successful treated with a variety of compression screw assemblies and intramedullary rods. The intramedullary rods are inserted into the narrow canal of the femur to immobilize the femur parts involved in the fracture. Typically, a single screw is inserted through the femur and the proximal end of the intramedullary rod. Alternatively, a second screw may be inserted through the femur and into the proximal end of the intramedullary rod to prevent rotation of, for example, the neck and head of the femur.

The nail sold under the trade mark Grosse-Kempf by Howmedica includes a threaded hole in the intramedullary rod for receiving an interlocking screw. The fully threaded screw cannot slide in order to permit the compression found in typical compression screw assemblies.

Another known device, which is disclosed in US-3433220 and is sold under the trade mark Zickel, is a solid intramedullary nail having a single proximal tri-flange cross-nail, which is inserted into the direction of the femoral head. The solid cross-section does not permit the nail to be introduced over a guide rod. Thus, the nail is prevented from being used for comminuted and distal fractures of the femur because the closed surgical technique cannot be practised. In addition, adequate compression cannot be achieved due to the requirement to lock the cross-nail.

A further known device, which is sold by Smith & Nephew Richards under the trade mark Russell-Taylor, requires a fully threaded locking screw and therefore does not permit sliding of the screw relative to the intramedullary rod.

Another known device, which is sold under the trade mark Gamma by Stryker-Howmedica, provides for sliding compression of the lag screw through the use of a smooth shaft. This device stops rotation of the lag screw by means of a setscrew through the proximal portion of the intramedullary nail.

A further known device, which is sold under the trade mark Ace by DePuy Orthopaedics Inc, provides for means of stopping rotation of the femoral head in an unstable fracture pattern by the use of a second threaded screw in the femoral head. The lag screw is permitted to rotate freely within the nail.

To promote and facilitate proper healing of bones, which have been fractured and have been repaired with femoral nails, the stability of the femur fracture is necessary to facilitate proper healing. Current products provide for the use of screws to the plate in the nails and to engage bone. These screws are limited in their placement and often times cannot be placed in the best location to reach stable bone. The limited ability to place nails in an intramedullary canal limit the ability to properly secure the bone and to provide the stability necessary to facilitate proper healing of the fractured long bone.

The present invention is directed to alleviate at some of the aforementioned concerns with orthopaedic fasteners.

The present invention is in the form of an intramedullary nail that contains a series of holes that allow for locking screws to be placed in various positions. The intramedullary nail can be locked statically or dynamically with the use of a dynamisation slot. The invention allows three separate screws to be placed through the nail at one time in two different planes. The ability to use multiple screws in multiple planes allows better stability to be achieved with the locking screws.

The nail design of the present invention provides for multiple screw fixations to be achieved in opposing planes for better fracture stabilization. In addition to using multiple planes, the screw creates a triangular geometry that aids in better axial and rotational stability.

The present invention provides for an intramedullary nail with one transverse hole, angulated crossing holes, and a static-dynamic slot for locking screw placement. The locking screws are placed through the nail in a combination that, if three screws are utilized, a (z) shape appears in the medial to lateral plane. When the three-screw construction is utilized, the added stability that is desired is accomplished. The screw configuration can be angulated so that a combination of one or two screws can be used depending on the fixation that is desired, based on a particular fracture pattern.

According to one embodiment of the present invention, there is provided an intramedullary nail for use in a medullary canal of a long bone. The nail includes a body defining a longitudinal axis and an external periphery of the body for fitting in the medullary canal of the long bone. The body has a first internal wall of the body defining a first opening through the body, the first opening defines a first opening centerline. The body has a second internal wall of the body defining a second opening through the body. The second opening defines a second opening centerline. The first opening centerline and the second opening centerline are oblique with respect to each other.

According to another embodiment of the present invention there is provided an intramedullary nail assembly for use in a medullary canal of a long bone. The nail assembly includes a nail defining a longitudinal axis and an external periphery of the nail for fitting in the medullary canal of the long bone. The nail has a first internal wall that defines first opening through the nail. This first opening defines first opening centerline. The nail has a second internal wall that defines second opening through the nail. This second opening defines second opening centerline. The first and second opening centerlines are oblique with respect to each other. The nail assembly also includes a first screw, which is slidably fitted to the first opening, and a second screw, which is also slidably fitted to the second opening.

The devices provided by the present invention can be used in a method for performing trauma surgery on a long bone. The method includes the step of providing an intramedullary nail. The nail defines a longitudinal axis and an external periphery of the nail for fitting in the medullary canal of the long bone. The nail has a first internal wall, which defines the first opening through the nail. The first opening defines a first opening centerline. The nail has a second internal wall of the nail defining a second opening through the nail. The second opening defines a second opening centerline. The first opening centerline and the second opening centerline are oblique with respect to each other.

The method can also include the steps of positioning the nail at least partially in the medullary canal and providing a first screw for cooperation with the long bone and for slidable cooperation with the first opening in the nail. The method further includes the steps of inserting the first screw through the cortical wall of the lesser trochanter of the long bone and inserting the first screw through the first opening. The method also includes the steps of inserting the first screw through the cortical wall of the greater trochanter of the long bone and providing a second screw for cooperation with the long bone and for slidable cooperation with the second opening in the nail. The method further includes the steps of inserting the second screw through the cortical wall of the long bone, inserting the second screw through the second opening, and inserting the second screw through the cortical wall of the long bone.

According to another embodiment of the present invention, there is provided an intramedullary nail for use in a medullary canal of a long bone. The nail includes a body defining a longitudinal axis and an external periphery of the body for fitting in the medullary canal of the long bone. The body has a first internal wall of the body defining a first opening through the body. The first opening defines a first opening centerline. The body has a second internal wall of the body defining a second opening through the body. The second opening defines a second opening centerline. The first and second opening centerlines are oblique with respect to each other. The first opening centerline and/or the second opening centerline are transverse to the longitudinal axis of said body.

According to yet another embodiment of the present invention there is provided a kit for use in repairing a fracture in a long bone. The kit includes a nail adapted for implantation in a medullary canal of the long bone. The nail defines a longitudinal axis and an external periphery of the nail for fitting in the medullary canal of the long bone. The nail has a first internal wall, which defines a first opening through the nail. This first opening defines first opening centerline. The nail has a second internal wall, which defines a second opening through the nail. This second opening defines a second opening centerline. The first and second opening centerlines are oblique with respect to each other. The first and/or second opening centerlines are transverse to the longitudinal axis of the nail. The kit includes a first screw, which is adapted to be slidably fittable with the first opening and similarly a second screw, which is adapted to be slidably fittable with the second opening.

The devices provided by the present invention can be used in a method for performing trauma surgery on a long bone. The method includes the step of providing an intramedullary nail. The nail defines a longitudinal axis and an external periphery of the nail for fitting in the medullary canal of the long bone. The nail has a first internal wall, which defines a first opening through the nail. This first opening defines the first opening centerline.

The nail has a second internal wall that defines a second opening through the nail. This second opening defines a second opening centerline. The first and second opening centerlines are oblique with respect to each other. At least one of the first or the second opening centerlines is transverse to the longitudinal axis of the nail. The method further includes the steps of positioning the nail at least partially in the medullary canal and providing a first screw for cooperation with the long bone and for slidable cooperation with the first opening in the nail. The method also includes the steps of inserting the first screw through the cortical wall of the lesser trochanter of the long bone and inserting the first screw through the first opening.

The method can further include the steps of inserting the first screw through the cortical wall of the greater trochanter of the long bone and providing a second screw for cooperation with the long bone and for slidable cooperation with the second opening in the nail. The method includes the step of inserting the second screw through the cortical wall of the long bone, inserting the second screw through the second opening, and inserting the second screw through the cortical wall of the long bone.

The technical advantages of the present invention include the ability to provide locking screws in various positions within the long bone. For example, according to one aspect of the present invention, an intramedullary nail for use in a medullary canal of a long bone is provided. The nail includes a body defining a longitudinal axis. The body has a first internal wall defining a first opening and a second internal wall defining a second opening. The first and second opening centerlines are oblique with respect to each other. Locking screws may be fitted in the first and second openings. Thus, the present invention provides for the ability to provide locking screws in various positions.

The technical advantages of the present invention further include the ability to allow two separate screws to be placed in one or two different planes. For example, according to another aspect of the present invention an intramedullary nail for use in the medullary canal of a long bone is provided. The nail includes a body defining a longitudinal axis. The body has a first wall defining a first opening and a second wall defining a second opening. The first and second openings are oblique with respect to each other. Each of the first and second openings may receive a screw. The longitudinal axis of the screws is thus oblique with respect to each other. Thus, the present invention provides for the ability to allow two separate screws to be placed in one or two different planes.

The technical advantages of the present invention further include the ability to allow for multiple screw fixations to be achieved in opposing planes for better fracture stabilization. For example, according to yet another aspect of the present invention, an intramedullary nail for use in a medullary canal of a long bone is provided. The nail includes a body having a first and second opening. The first and second openings are oblique with respect to each other. Each of the respective holes may serve to receive a screw. Thus, the present invention provides for multiple screw fixations to be achieved in opposing planes for better fracture stabilization.

The technical advantages of the present invention also include the ability to place screws in multiple planes to treat unstable femur fractures. For example, according to another aspect of the present invention, an intramedullary nail for use in a femur is provided. The nail includes a body having first and second holes. The first and second holes are oblique to each other or are in multiple planes. Thus, the present invention provides for the ability to provide a nail that can place screws in multiple planes to treat unstable femur fractures.

The technical advantages of the present invention include the ability to use three screws to provide for a triangular geometry that aids in better axial and rotational stability for the patient. For example, according to yet another aspect of the present invention, an intramedullary nail for use in the medullary canal of a long bone is provided. The nail includes a body. The body has a first, second and third hole. Each of the three holes is positioned such that they provide for a triangular geometry. Thus, the present invention provides for the use of three screws that provide a triangular geometry that aids in better axial and rotational stability.

The technical advantages of the present invention also include the ability to prevent trochanteric to trochanteric and femoral neck fixation with the same nail. For example, according to another aspect of the present invention, an intramedullary nail for use in the medullary canal of a long bone is provided. The nail includes a first transverse opening, a second spaced apart transverse opening, and an oblique opening. The transverse opening centerlines and the oblique centerlines are oblique with respect to each other. Thus, the present invention provides for trochanter-to-trochanter and femoral neck fixation within the same nail.

Embodiments of the invention will now be described by way of example with reference to the accompanying drawings, in which:
FIG. 1 is a partial anterior/posterior view of a intramedullary nail assembly implanted in a patient in accordance with an embodiment of the present invention with a screw directed toward the neck, two transverse screws, and an opening for a fourth screw;
FIG. 1A is a partial anterior/posterior view of a nail with a longitudinal groove in accordance with another embodiment of the present invention;
FIG. 2 is an anterior/posterior view of the intramedullary nail of the intramedullary nail assembly of FIG. 1;
FIG. 3 is a medial/lateral view of the intramedullary nail of FIG. 2;
FIG. 3A is a cross section view of FIG. 2 along the line 3A-3A in the direction of the arrows;
FIG. 3B is an enlarged partial medial/lateral view of FIG. 3 showing the chamfer on the distal end of the nail in greater detail;
FIG. 3C is an enlarged partial anterior/posterior view of the nail of FIG. 3 showing the chamfer in greater detail;
FIG. 4 is an enlarged partial medial/lateral view of the proximal end of the intramedullary nail of FIG. 2;
FIG. 4A is an end view of FIG. 4;
FIG. 5 is an enlarged partial anterior/posterior view of the proximal end of the intramedullary nail of FIG. 2;
FIG. 6 is a cross sectional view of FIG. 2 along the line 6-6 in the direction of the arrows;
FIG. 7 is a partial anterior/posterior view of the intramedullary nail assembly of FIG. 1 implanted in a femur with a screw directed toward the neck and two transverse screws;
FIG. 8 is a partial anterior/posterior view of the intramedullary nail assembly of FIG. 1 with two transverse screws;
FIG. 9 is a partial anterior/posterior view of the intramedullary nail assembly of FIG. 1 with a screw directed toward the greater trochanter and two transverse screws;
FIG. 10 is a partial perspective view of the nail assembly of FIG. 1 with a partially threaded screw extending from the lesser trochanteric to the greater trochanteric as well as with a transverse partially threaded screw to form a nail assembly according to another embodiment of the present invention;
FIG. 11 is a plan view of a cortical screw for use with the nail assembly of FIG. 1, for example as a transverse screw or a lesser trochanter to greater trochanter screw;
FIG. 12 is a plan view of a cancellous screw for use with the nail assembly of FIG. 1, for example as a femoral neck screw;
FIG. 12A is a plan view of another cancellous screw for use with the nail assembly of FIG. 1, for example as a distal screw;
FIG. 13 is a partial anterior/posterior view of a intramedullary nail assembly implanted in a patient in accordance with another embodiment of the present invention with a cannulated screw directed toward the neck and two transverse screws;
FIG. 14 is a plan view of a lag screw for use in the intramedullary nail assembly of FIG. 13;
FIG. 15 is a cross sectional view of the lag screw of FIG. 14 along the line 15-15 in the direction of the arrows;
FIG. 16 is an enlarged partial view of the box type thread form for the lag screw of FIG. 14;
FIG. 17 is a partial view of a standard thread form for an alternate lag screw for use with an alternate embodiment of the intramedullary nail assembly of the present invention;
FIG. 17A is a partial view of a V-shaped thread form for an alternate lag screw for use with an alternate embodiment of the intramedullary nail assembly of the present invention;
FIG. 17B is a partial view of a square-shaped thread form for an alternate lag screw for use with an alternate embodiment of the intramedullary nail assembly of the present invention;
FIG. 17C is a partial view of a truncated V-shaped thread form for an alternate lag screw for use with an alternate embodiment of the intramedullary nail assembly of the present invention;
FIG. 17D is a partial view of a reverse box thread form for an alternate lag screw for use with an alternate embodiment of the intramedullary nail assembly of the present invention;
FIG. 17E is a partial view of yet another alternate thread for use with another alternate embodiment of the present invention;
FIG. 18 is a partial anterior/posterior view of a intramedullary nail assembly implanted in a patient in accordance with yet another embodiment of the present invention with a screw directed toward the neck and two transverse screws;
FIG. 19 is a partial medial/lateral view of the nail of the intramedullary nail assembly of FIG. 18 showing the proximal portion of the nail in greater detail;
FIG. 20 is a partial anterior/posterior view of the intramedullary nail assembly of FIG. 18 showing its use with only the two transverse screws, one being fully threaded and one being partially threaded;
FIG. 21 is a partial anterior/posterior view of a intramedullary nail assembly in accordance with another embodiment of the present invention with a partially threaded screw extending from the lesser trochanteric to the greater trochanteric as well as with a transverse partially threaded screw and with a transverse fully threaded screw to form the nail assembly;
FIG. 22 is a partial medial/lateral view of the nail of the intramedullary nail assembly of FIG. 21 showing the proximal portion of the nail in greater detail;
FIG. 23 is a partial anterior/posterior view of the intramedullary nail assembly of FIG. 21 in position in the femur showing its use with only one fully threaded screw extending from the lesser trochanter to the greater trochanter;
FIG. 24 is a partial anterior/posterior view of the intramedullary nail assembly of FIG. 21 in position in the distal femur showing its use with use with only the two transverse screws, both being fully threaded;
FIG. 25 is an anterior/posterior view of the intramedullary nail assembly implanted in a patient in accordance with a further embodiment of the present invention with a nail adapted for entry in the lesser trochanter;
FIG. 26 is a medial/lateral view of the intramedullary nail assembly of FIG. 25;
FIG. 26A is an enlarged partial medial/lateral view of FIG. 26 showing the chamfer of the distal end in greater detail;
FIG. 26B is an enlarged partial anterior/posterior view of the chamfer in the distal end of the nail;
FIG. 27 is a partial anterior/posterior view of the intramedullary nail assembly of FIG. 25 in position in the femur showing its use with only one fully threaded screw extending into the neck of the femur;
FIG. 28 is a plan view of a kit for use in performing trauma surgery in accordance with yet another embodiment of the present;
FIG. 29 is a flow diagram of a method of performing trauma surgery in accordance with yet another embodiment of the present; and
FIG. 30 is a flow diagram of another method of performing trauma surgery in accordance with another embodiment of the present invention.

Corresponding reference characters indicate corresponding parts throughout the several views. Like reference characters tend to indicate like parts throughout the several views.

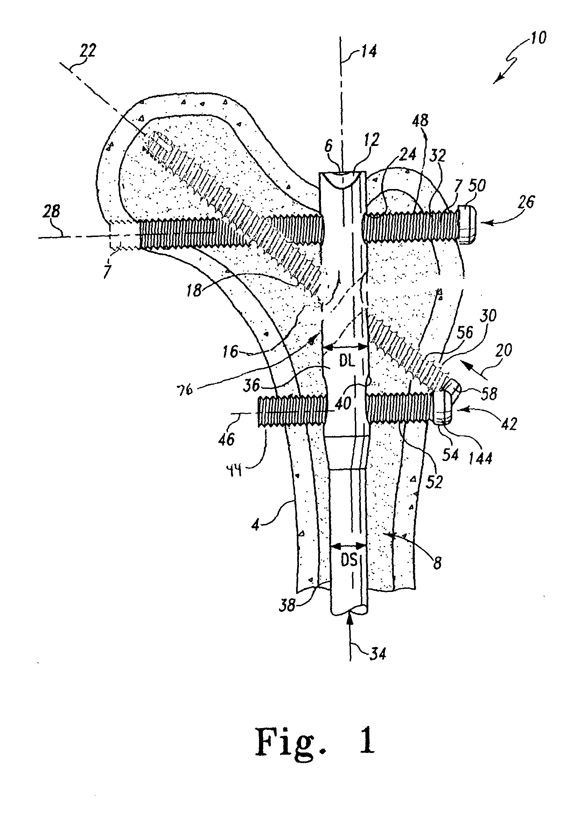

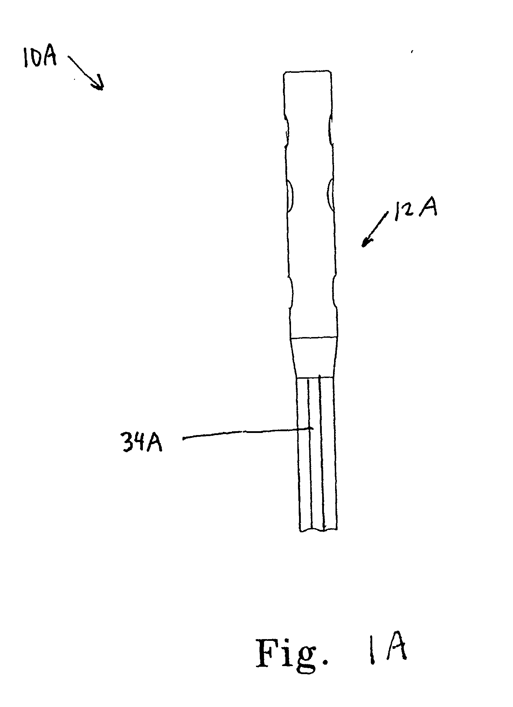

Referring to the drawings, FIG. 1 shows an intramedullary nail assembly 10 for use in a medullary intramedullary canal 6 of a long bone 4. The intramedullary canal assembly 10 includes an intramedullary nail 12. The nail 12 defines a longitudinal axis 14 and an external periphery 16 of the intramedullary nail 12. The external periphery 16 is sized for fitting within the intramedullary canal 6 of the long bone 4. The nail 12 has a first internal wall 18. First internal wall 18 defines a first opening 20 through the nail 12. The first opening 20 defines a first opening centerline 22. The nail 12 further has a second internal wall 24. The second internal wall 24 defines a second opening 26 through the nail 12. The second opening 26 defines a second opening centerline 28. The first opening centerline 22 and the second opening centerline 28 are oblique with respect to each other. The intramedullary nail assembly 10 further includes a first screw 30. The first screw 30 may be slidably fitted to the first opening 20. The intramedullary nail assembly 10 also includes a second screw 32. The second screw 32 may be slidably fitting to the second opening 26.

The nail 12 may have any suitable shape capable of being inserted into the intramedullary canal 6 of the femur 4. The intramedullary canal 6 may be solid or may, as is shown in FIG. 1, be cannulated or include a longitudinal opening 34. The nail 12 may have any shape. It may, for simplicity, have a generally round or circular cross section. The nail 12 may have a uniform cross section or may, as is shown in FIG. 1, have a larger diameter in the condylar area of the nail and, in particular, the proximal condylar area of the nail.

For example as shown in FIG. 1 the nail 12 includes a proximal portion 36 and a distal portion 38. The proximal portion 36 has a larger diameter DL while the distal portion 38 has a smaller diameter DS. The larger diameter DL of the proximal portion 36 serves to provide support for the screws located in the proximal portion 36 of the nail 12.

As shown in FIG. 1, the nail 12 may further include a third internal wall 40 defining a third opening 42. A third fastener 44 in the form of, for example, a screw may be fitted in the third opening 42. The third opening 42 may define a third opening centerline 46. The screws 30, 32 and 44 may be any type which are capable of cooperating with bone.

For example, the screws 30, 32 and 44 may be in the form of cancellous or cortical screws. The second opening 26 and the third opening 42 as shown in FIG. 1 may be transverse.
The second screw 32 and the third screw 44, in that they fit in the transverse openings, are preferably cortical screws so that they can engage opposed cortical walls of the femur 4.

For example, as shown in FIG. 1, the second screw 32 includes a threaded body 48 composed of cortical threads. The second screw 32 further includes a head 50. The second screw 32 extends as shown in FIG. 1 from the cortical wall 7 of the femur 4 across cancellous bone 8 through the second opening 26, through the cancellous bone 8 and again into cortical wall 7. The support of the second screw 32 by the opposed cortical wall 7 provides ample support for the second screw 32. The head 50 of the second screw 32 rests against the outer surface of the cortical wall 7.

Similarly the third screw 44 is preferably in the form of a cortical screw. The third screw 44 includes a cortical threaded body 52 and a head 54. The cortical threaded body 52 passes from the cortical wall 7 through cancellous bone 8, through the third opening 42, through additional cancellous bone 8 and into the opposed cortical wall 7. The head 54 of the third screw 44 rests against the cortical wall 7 of the femur 4.

The first screw 30 may be a cancellous or a cortical screw. Since the first screw 30 passes into the neck of the femur and maybe used to secure a fracture in the head of the femur, the first screw 30 may be a cancellous screw. The first screw 30, as is shown in FIG. 1, is in the form of a cancellous screw. The first screw 30 includes a threaded cancellous body 56 and a head 58. The first screw 30 passes through cortical wall 7, cancellous bone 8, first opening 20, and into additional cancellous bone 8.

Referring now to FIG. 1A, another embodiment of the present invention is shown as nail assembly 10A, which includes nail 12A. The nail 12A has a shape somewhat different than the nail 12 of FIG. 1, in that nail 12A of FIG. 1A includes a groove 34A formed in the nail 12A and serves the same general purpose as longitudinal opening 34 of the nail 12 of FIG. 1.

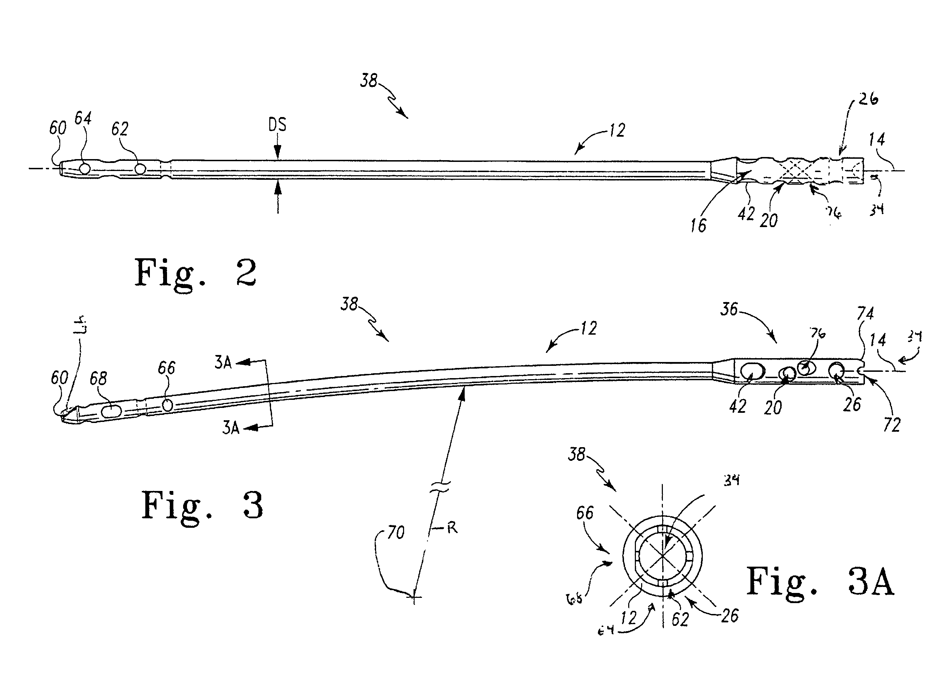

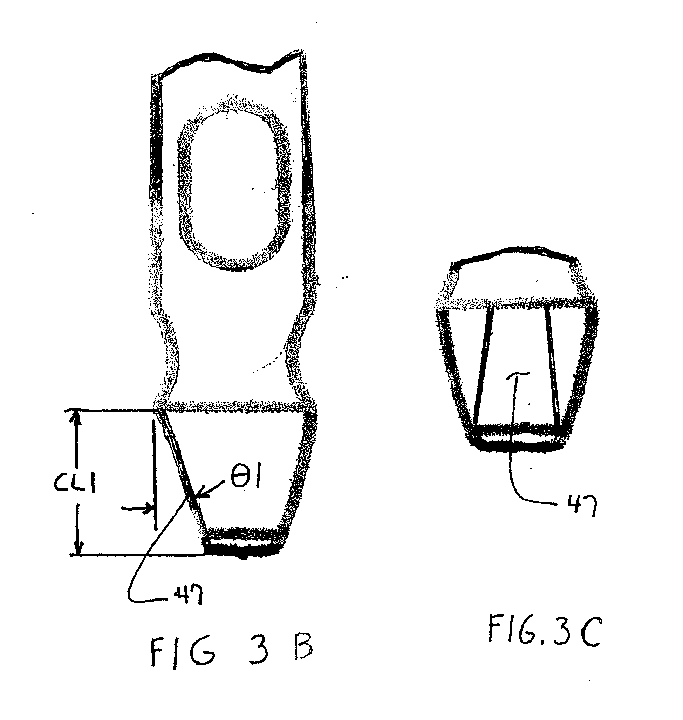

The nail 12 may, as shown in FIG. 3 include a relief surface such as a flat surface for example a chamfer 47 for assisting in leading the curved nail 12 into the medullary canal of the long bone, for example the femur. It should be appreciated that the chamfer may have a surface that is not flat, for example arcuate, for example a portion of a sphere or a cylinder.

Referring now to FIG. 3B, the chamfer 47 is shown in the medial/lateral view with chamfer 47 shown on the side of the distal tip opposed to the origin 70 of the curved portion of the nail 12. The chamfer may be defined by angle 1 from the longitudinal periphery of the nail 12. The chamfer may be further defined by chamfer length CL1 from the distal end of the nail 12.

Referring now to FIG. 3, the chamfer 47 is shown in the anterior/posterior view with chamfer 47 shown at distal tip. It should be appreciated that the tip may be larger or smaller than shown.

Referring now to FIGS. 2 and 3, the intramedullary nail 12 of the nail assembly 10 of FIG. 1 is shown. The nail 12 includes the distal portion 38 and the proximal portion 36. The distal portion is fully shown in FIGS. 2 and 3. The distal portion as shown in FIG. 2 is cylindrical and defined by diameter DS. The anterior/posterior view of the nail 12 of FIG. 2 is straight and extends along longitudinal axis 14.

The nail 12 includes a series of holes or openings adjacent distal end 60 of the nail 12. As can be seen in FIGS. 2 and 3, the end 60 has a generally tapered shape to assist in the insertion of the nail 12 into the medullary canal. The distal portion 38 of the nail 12 near the end 60 includes a plurality of holes or openings for securing the distal portion 38 of the nail 12 in the long bone or femur 4.

For example, and as shown in FIGS. 2 and 3, the anterior/posterior view of the nail 12 of FIG. 2 shows a first distal opening 62 and a second distal opening 64. The distal openings 62 and 64 as shown in FIG. 2 are transverse or perpendicular to longitudinal axis 14.

Referring now to FIG. 3, the distal end 38 of the nail 12 may include additional holes near the end 60 of the nail 12. For example, and as shown in FIG. 3 the nail 12 includes a third distal opening 66 which is transverse to the longitudinal axis 14 of the nail 12 and a fourth distal opening 68 which is also transverse or perpendicular to the longitudinal axis 14 of the nail 12. The cross section of the distal openings 62, 64, 66 and 68 may have any shape and for simplicity, and as shown in FIGS. 2 and 3, the nail openings may be circular in cross section. For example the distal openings 62, 64 and 66 are shown circular in cross section. The fourth distal opening 68 as shown in FIG. 3 has an oval cross section.

Referring now to FIG. 3, the medial/lateral shape of the nail 12 conforms to the medullary canal of a femur. The medial/lateral plane of the femur is curved and, as such, the nail 12 is curved in the distal portion 38. As shown in FIG. 3 the distal portion 38 of the nail 12 has a bend along longitudinal axis 14 defined by radius R extending from origin 70.

Referring to FIG. 3, the proximal portion 36 of the nail 12 may include a notch 72 for assisting in inserting the nail 12 and for orienting the nail 12, as well as, a chamfer 74 for providing clearance for soft tissue in the anatomy.

Referring now to FIG. 3A, the distal portion 38 of nail 12 has a hollow circular cross section. The first distal opening 62 may, as shown, be perpendicular to the third distal opening 66. The central opening or cannula 34 is located in the nail 12.

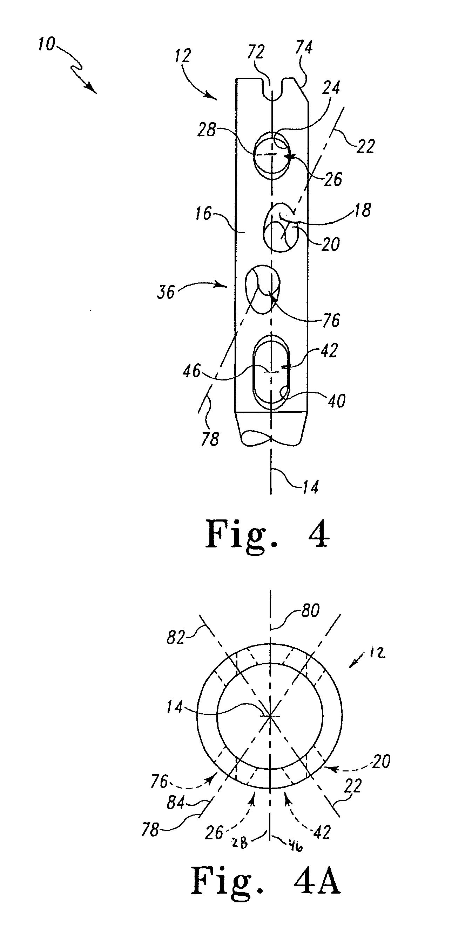

Referring now to FIG. 4 and FIG. 4A, the proximal portion 36 of the nail 12 may, as is shown in FIG. 4 and 4A, further include a fourth proximal opening 76 defining a fourth opening centerline axis 78. The fourth opening 76 like the first opening 20 is skewed or oblique with respect to the plane, which defined the second and third openings, 26 and 42.

For example and as shown in FIG. 4A, centerline 28 of the second opening 26 and the centerline 46 of third opening 42 define a first opening plane 80. As shown in FIG. 4A, the plane 80 is coplanar with longitudinal centerline 14 of the nail 12. Centerline 22 of the first opening 20 defines a second plane 82 that intersects plane 80 at longitudinal axis 14 of the nail 12.

Similarly, centerline 78 of the fourth opening 76 defines a third plane 84 that is positioned angularly from the first plane 80 and the second plane 82 and intersects the first plane 80 and the second plane 82 at longitudinal centerline 14. By providing the first opening 20 and the second opening 26 at different planes than the second opening 26 and the third opening 42, a plurality of screws can be placed in an intersecting arrangement within a nail 12, simultaneously, by having them pass through different portions of the nail and not intersect or interfere with each other.

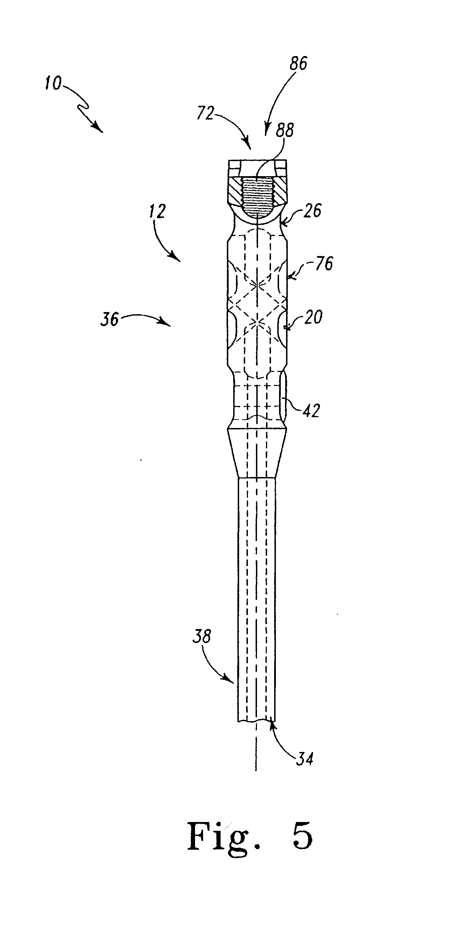

Referring now to FIG. 5, the longitudinal opening or cannula 34 of the nail 12 may include a counterbore 86 on which internal threads 88 may be formed. The internal threads 88 may cooperate with a threaded fastener (not shown), which may engage the screws to lock the screws in position in the nail.

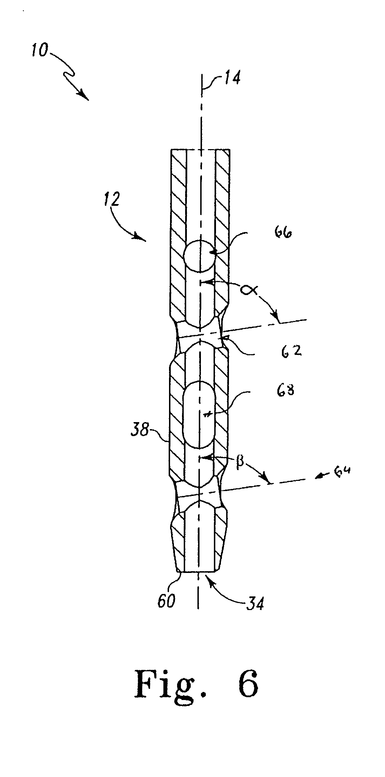

Referring now to FIG. 6, distal portion 38 of the nail 12 near distal end 60 is shown in greater detail. As described earlier, the distal portion 38 of the nail 12 includes a series of openings, for example, as shown in FIG. 6, four spaced apart openings. Two of the openings are oriented in the medial/lateral plane and two of the openings are oriented in the anterior/posterior plane. These openings provide for distal fixation of the nail by fasteners.

The openings may be normal or perpendicular to longitudinal axis 14 of the nail 12. For example, third opening 66 is normal or perpendicular to longitudinal axis 14. The third opening 66 has a generally cylindrical shape. The fourth opening 68 is also perpendicular to the longitudinal axis 14 of the nail 12 except that the fourth opening 68 is oval. The openings in the nail may also be skewed or not perpendicular to the longitudinal opening 34. For example, first opening 62 intersects the longitudinal axis at an acute angle while the second opening 64 intersects the longitudinal axis at an acute angle.

It should be appreciated that the distal openings 62, 64, 66 and 68 pass through both external walls of the cannulated nail 12. It should also be appreciated that the four openings in the proximal portion 36 of the nail 12 likewise pass through both external walls of the cannulated nail 12. For example, the first opening 20, second opening 26, third opening 42 and fourth opening 76 all have an opening on each of the exterior walls of the nail 12 such that the opening passes through both external walls of the nail 12.

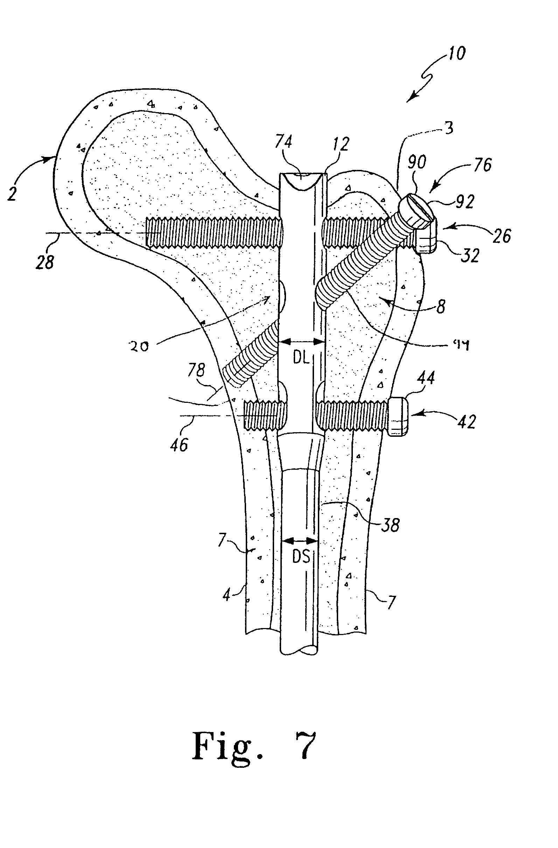

Referring now to FIG. 7, the nail assembly 10 of the present invention is shown for use with the greater to lesser trochanter attachment of the intramedullary nail. As shown in FIG. 7, the nail assembly 10 is shown installed in a left femur.

The nail assembly 10 includes the nail 12 as well as second screw 32, which is positioned in second opening 26 and third screw 44, which is positioned in third opening 42. The nail assembly 10 as shown in FIG. 7, further includes fourth screw 90 which is positioned in fourth opening 76 and positioned along fourth opening centerline 78.

According to the present invention, the fourth screw 90 may be installed in the nail 12 while the second screw 32 and the third screw 44 are installed in the same nail 12. The ability to place the fourth screw 90 in the nail 12 while the second screw 32 and third screw 44 are also installed in the nail 12 is possible because the fourth screw 90 is positioned in a different plane than the second screw 32 and the third screw 44.

For example, and referring again to FIG. 4A, the second opening 26 and the third opening 42 define first plane 80 while the fourth opening 76 defines the third plane 84.

Referring again to FIG. 7, by positioning the fourth opening 76 and fourth screw 90 in a plane different from the second opening 26 and third opening 42, the fourth screw 90 can be positioned as shown. The second screw 32, third screw 44 and the fourth screw 90, form a z-shaped pattern, which provides improved rigidity, strength and stability for the intramedullary nail assembly 10.

The fourth screw 90 as shown in FIG. 7 may be a cortical screw and may as shown in FIG. 7 include a head 92 and a shaft 94 having cortical threads. The screw 90 is installed by passing it through cortical wall 7 through cancellous bone 8, through the fourth opening 76 in the nail 12, through additional cancellous bone 8, and into the second cortical wall 7. The head 92 of the screw 90 seats against the outer surface of the cortical wall 7 of the femur 4.

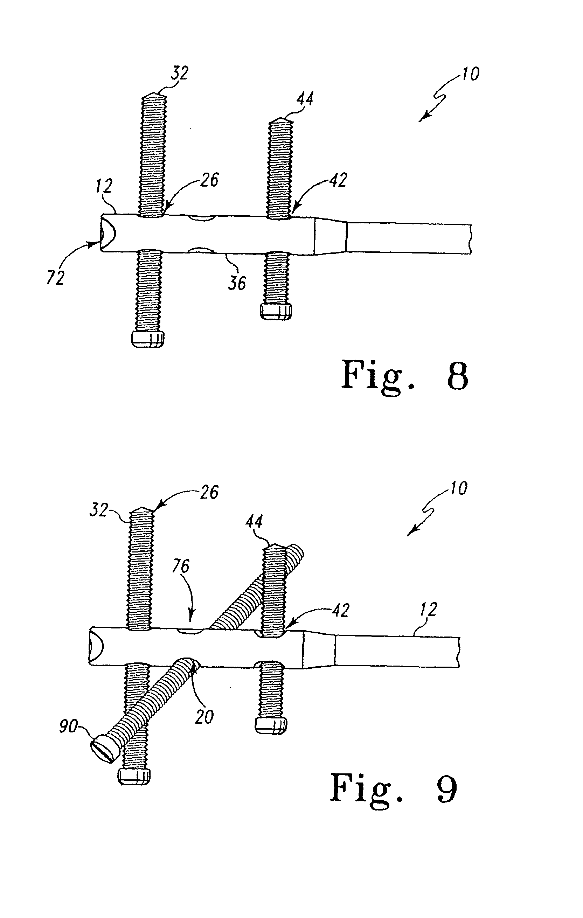

Referring now to FIG. 8, yet another configuration for the nail assembly 10 of the present invention is shown. The nail assembly 10 of FIG. 8 includes the nail 12 as well as second screw 32, which is positioned in opening 26 and third screw 44, which is positioned in third opening 42. The nail assembly 10 as shown in FIG. 8 may be used in both the right and left femur and may have the proximal portion 36 of the nail 12 positioned in the proximal condylar portion. The proximal portion 36 of the nail 12 may, alternatively, be positioned in the distal condyle of the femur. If the proximal portion 36 of the nail 12 is positioned in the distal condyle, the nail assembly 10 serves as a retrograde nail.

Referring now to the configuration as shown in FIG. 9, the nail assembly 10 is utilized on a right femur. When the nail assembly 10 of the present invention is used in a right femur, rather than a left femur, as shown in FIG. 1 and FIG. 7, the function of the first opening 20 and the fourth opening 76 are reversed. For example, the nail assembly 10 of FIG. 9 includes the nail 12 as well as second screw 32, which is fitted in second opening 26 and third screw 44, which is fitted in third opening 42. The nail assembly 10 of FIG. 9 further includes fourth screw 90, which is fitted into first opening 20. The fourth screw 90 is utilized to secure the greater trochanter 3 to the lesser trochanter 5 (see FIG. 7).

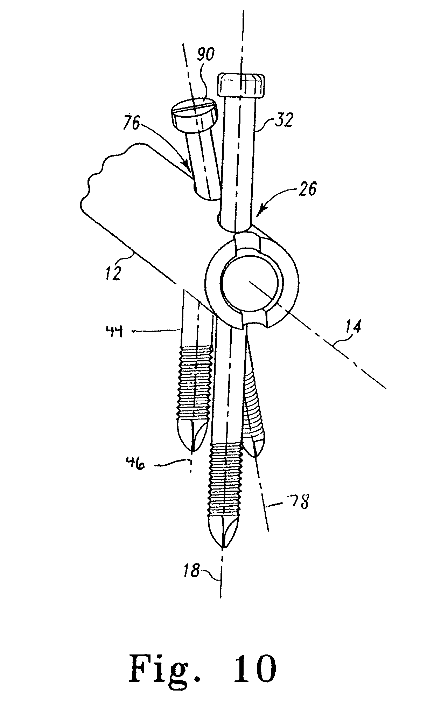

Referring now to FIG. 10, second screw 32, third screw 44 and fourth screw 90 are shown installed into the nail 12. The configuration, as shown in FIG. 10, is the same as the configuration as shown in FIG. 7. FIG. 10 illustrates the positioning of second screw 32 and fourth screw 90 in different planes. By providing the second screw 32 and the fourth screw 90 in different planes, the oblique screw 90 and the transverse screw 32, as used as transverse screw 44, may be installed at the same time.

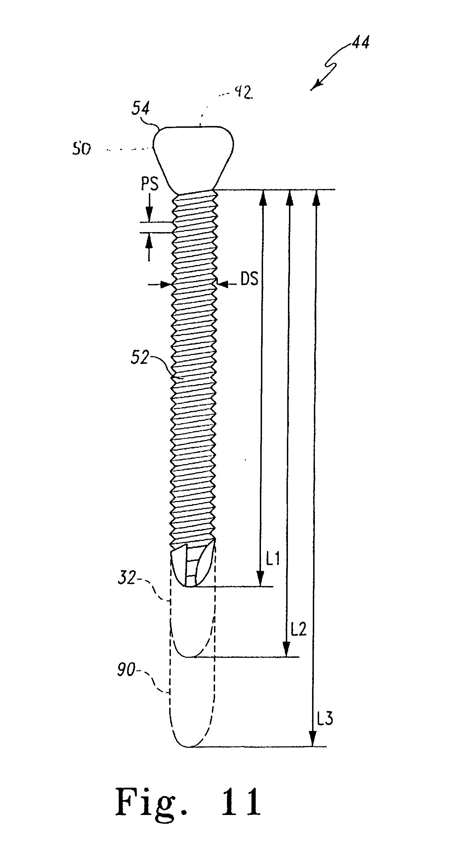

Referring now to FIG. 11, a cortical screw for use with the nail assembly of the present invention is shown. The cortical screw as shown in FIG. 7 may be used for example as third screw 44. The third screw 44 has a length L1, a diameter DS and a pitch PS. The diameter DS is chosen to slidably fit in third opening 42. The third screw 44 has a length L1 such that the third screw 44 may have head 54 of the third screw 44 rests against cortical wall 7 of the femur 4 and extend through the third opening 42 and be secured in the opposed cortical wall 7 of the femur 4 (see FIG. 7). The cortical screw 44 has a screw pitch PS, which is chosen to provide for optimum engaging strength for the screw 44 with the cortical bone.

The third screw 44, as well as the second screw 32, the first screw 30 and the fourth screw 90 may all be cortical screws. Preferably and as shown in FIGS. 1 and 7 the third screw 44, the second screw 32 and the fourth screw 90 are preferably cortical screws.

The second screw 32 as shown in dashed lines in FIG. 11 has a length L2 sufficient to permit the second screw 32 to have second screw head 50 rest upon the cortical wall of the femur and extend into a opposed cortical wall of the femur.

The fourth screw 90 is shown in phantom in FIG. 11. The fourth screw 90 is also a cortical screw and has a screw length L3 sufficient to have the head 92 of the fourth screw 90 rest against the cortical wall of the femur and the opposed end of the cortical screw 90 extend into the opposed cortical wall of the femur. The second screw 32 and the fourth screw 90 may have a diameter sufficient to slidably fit into the second opening 26 and fourth opening 76, respectively. The second screw 32 and the fourth screw 90 may have a pitch diameter PS identical to that of third screw 44.

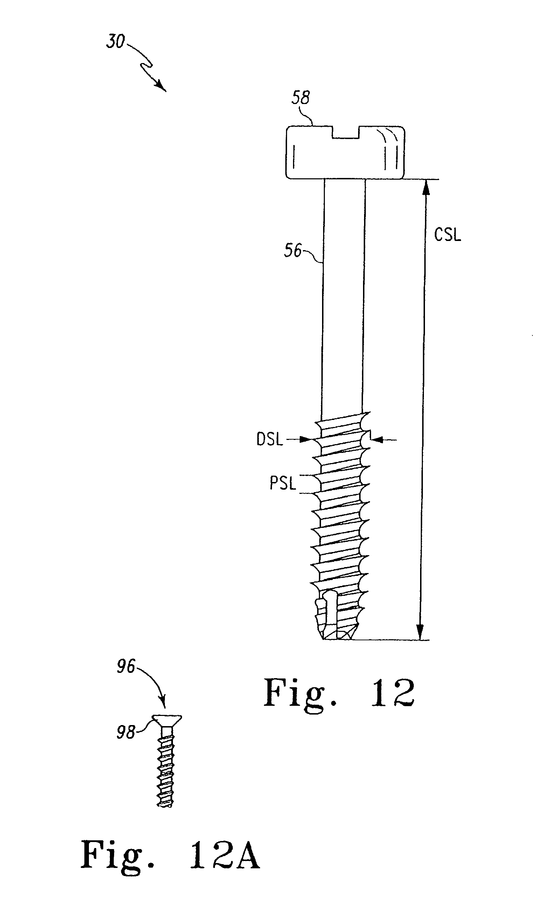

Referring now to FIG. 12, a cancellous screw is shown. The cancellous screw as shown in FIG. 12 may be utilized in any of the openings of the nail assembly 10 of the present invention. The cancellous screw as shown in FIG. 12 may be in the form of first screw 30 of the nail assembly 10 of FIG. 1. The cancellous screw 30 is designed to have the head 58 rests against the outer cortical wall of the femur and have a body 56 which includes cancellous threads which engage with cancellous bone. The cancellous screw 30 has a screw length CSL and a diameter DSL sufficient to slidably fit within the first opening 20 of the nail 12 (see FIG. 1). The cancellous screw 30 has a pitch PSL sufficient to engage the cancellous bone.

Referring now to FIG. 12A, small cortical screw 96 is shown. The small cortical screw 96 may be fitted into any of the first opening 62, second opening 64, third opening 66 and fourth opening 68 of distal portion 38 of the nail 12 (see FIG. 6). The small cortical screw 96 may include a head 98 for engagement with the outer cortical wall of the femur.

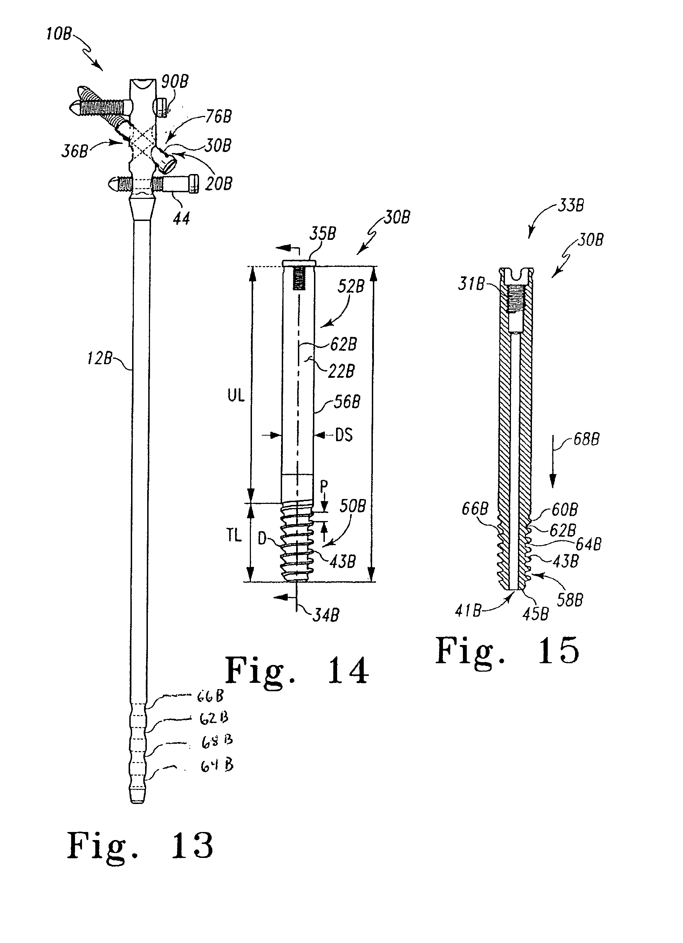

Referring now to FIG. 13, yet another embodiment of the present invention is shown as nail assembly 10B. The nail assembly 10B is similar to the nail assembly 10 of FIGS. 1 through 12 except that the nail assembly 10B includes a first screw 30B that is adapted for sliding compression in first opening 20B of nail 12B. The screw 30B may be larger than screw 30 of nail assembly 10 of FIG. 1. The opening 20B of nail 12B may thus be larger than opening 20 of nail 12 (see FIG. 1). Since the first opening 20B and fourth opening 76B are both used with first screw 30B and fourth screw 90B depending on whether the nail 12B is in the right or left femurs, the fourth opening 76B and the first opening 20B may have the same diameter.

The nail 12B may include a proximal portion 36B similar to the proximal portion 36 of the nail 12 of FIG. 1. The nail 12B may further include a distal portion 38B similar to the distal portion 38 of the nail 12 of FIG. 1. The distal portion 38B may include a first opening 62B, a second opening 64B, a third opening 66B, and a fourth opening 68B. The openings in the distal portion 38B may be similar or identical to the openings in the distal portion 38 of the nail 12 of FIG. 1.

Referring now to FIG. 14, the screw 30B may further include a removal feature 31B in the form of, for example, internal threads formed in the small counter bore 33B formed in the longitudinal opening 41B adjacent the second end 35B of the screw 30B. The screw 30B may further include a large counter bore 37B extending from the second end 35B of the lag screw 30B and concentric with the small counter bore 33B as well as with the longitudinal opening 41B.

Referring to FIG. 14, the screw 30B may further include a plurality of threads 43B formed on the shank periphery of shank 56B of the screw 30B. The threads 43B may, as shown in FIG. 14, have a non-uniform cross-section, as disclosed in US-A-2006/0106389.

Referring again to FIG. 14, the periphery 22B of the shank 56B of the screw 30B includes a first portion 50B into which the threads 43B are formed. It should be appreciated that the first portion 50B may extend along the longitudinal opening 34B of the screw 30B from the first end 39B to second end 35B of the screw 30B. It should also be appreciated and, as is shown in FIG. 14, that the periphery 22B may include a second portion 52B. The second portion 52B of periphery 22B of the shank 56B may define a smooth surface 62B. As is shown in FIG. 14, the periphery 22B of the shank 56B may be generally cylindrical and defined by a diameter, for example, DS.

The screw 30B as is shown in FIG. 14, is generally cylindrical and defined by a thread diameter D and an overall length L. The shank 56B of the screw 30B includes the first portion 50B which include threads 43B and the second portion 52B having the smooth surface 62B. The overall length L of the screw 30B is divided into a thread TL and a smooth or unthreaded length UL. The thread length TL defines the first portion 50B and the smooth length UL defines the second portion 52B. The thread length TL may, for example, be a portion of, for example, 20 to 40% of the overall length L of the shank 56B. It should be appreciated that the smooth length UL is preferably a sufficient length such that the second portion 52B of the screw 30B may be positioned in the opening 20B of the intramedullary nail 12B (see FIG. 13) to permit compression of the bone fracture of the femur.

The threads 43, as is shown in FIG. 14, may advance spirally around the periphery 22B of the shank 56B of the screw 30B. The threads 43B may be defined by a pitch P defining spacing along longitudinal opening 34B between adjacent threads. The threads 43B may advance spirally around the longitudinal opening 34B in either a right or a left hand spiral configuration. The threads may, as is shown in FIG. 14, be of a single lead type but may alternatively be double lead configuration or a triple lead configuration.

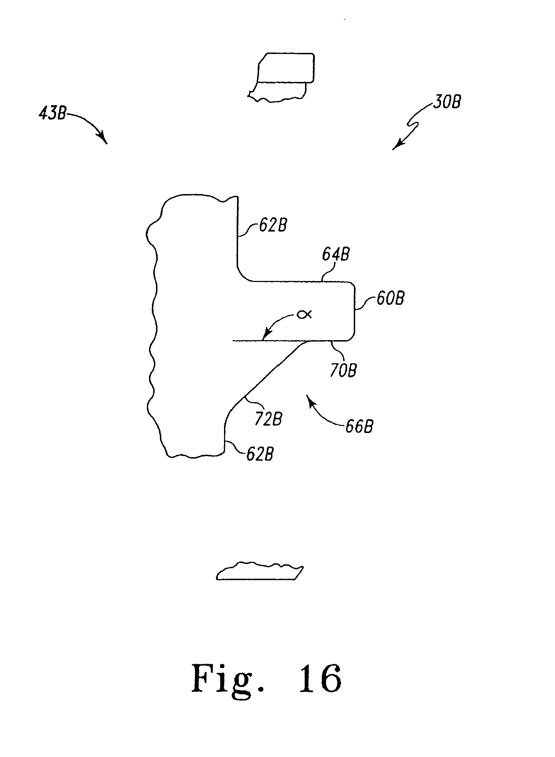

Referring now to FIG. 15, the threads 43B may have any suitable shape or thread form. For example and as shown in FIG. 15, the threads 43B may have a combination box and tapered configuration. For example and is shown in FIG. 15, the threads 43B may have any suitable shape or profile 58B. For example, and is shown in FIG. 15, the profile 58B may include a crest 60B and opposed root 62B. A trailing surface 64B is positioned between the crest 60B and the root 62B adjacent the second end 45B of the screw 30B while the leading edge 66B is positioned between the crest 60B and root 62B adjacent the first end 35B of the screw 30B.

As shown in FIG. 15, the leading edge 66B and the trailing edge 64B may be configured to provide for less force to assemble in the direction of arrow 68B than to disassemble in the direction opposed to arrow 68B. Such ease of assembly and difficulty in disassembly may be accomplished as is shown in FIG. 15 by providing the trailing edge 64B with a configuration that is normal or perpendicular to the root 62B and the crest 60B while providing the leading edge 66B with chamfered or angled surface or, as is shown in FIG. 15, or with a partially angled surface between the crest 60B and the root 62B.

Referring now to FIG. 16, the threads 43B are shown in greater detail. The threads 43B of the screw 30B may, as is shown in FIG. 16, include the leading edge 66B such that the leading edge 66B includes normal or perpendicular portion 70B as well as an angled portion 72B. The angled portion 72B provides for reduced force to assemble the screw 30B into the long bone or femur 4. The perpendicular portion 70B and the angled portion 72B may define an angle there between. To minimize stress, the crest 60B, the root 62B, trailing edge 64B, and leading edge 66B may include arcuate portions there between to minimize the stress.

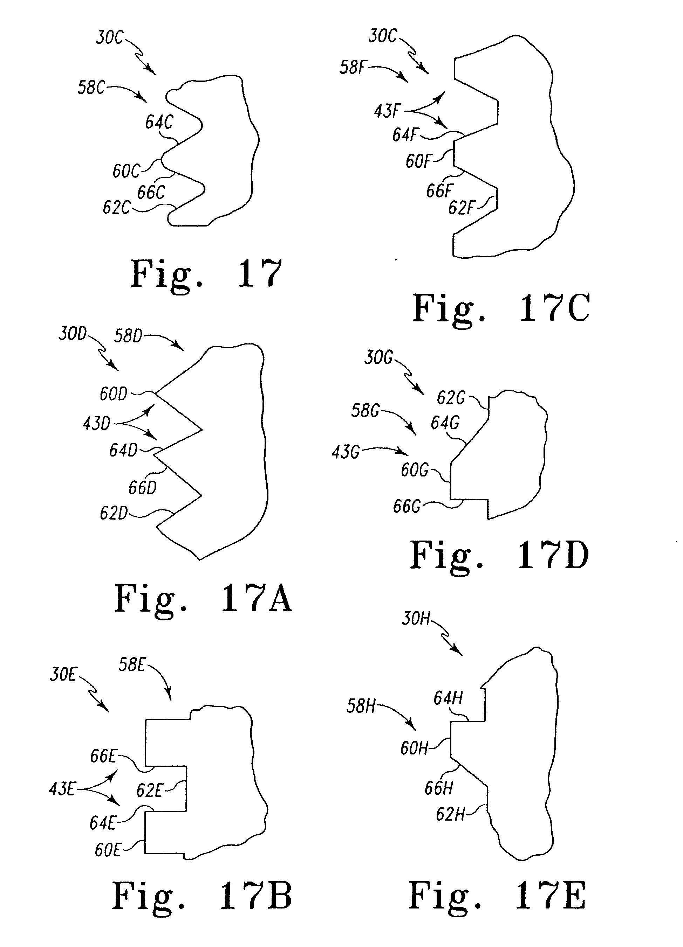

Referring now to FIG. 17-17E, an alternative profile configuration for threads of the screw of the nail of the present invention is shown. Referring now to FIG. 17, profile 58C is shown which includes arcuate roots and crest. For example, and is shown in FIG. 17, the profile 58C of the screw 30C includes an arcuate crest 60C to which the trailing angled surface 64C extends. The leading edge 66C extends likewise from the arcuate crest 60C. The profile 58C further includes an arcuate root 62C, which connects with trailing surface 64C and leading surface 66C.

Referring now to FIG. 17A, yet another profile for threads for the screw of the present invention is shown as screw 30D includes threads 43D having a profile 58D which include generally v-shaped threads 43D. The profile 58D includes trailing surface 64D and leading surface 66D. The root 62D and the crest 60D, as shown in FIG. 17A, are minimal.

Referring now to FIG. 17B, yet another profile of threads for a screw according to the present invention is shown. For example and is shown in FIG. 17B, the screw includes threads 43E having a profile 58E that is blocked or rectangular. The profile 58E includes parallel and spaced apart root 62E and crest 60E. The profile 58E includes a trailing surface 64E, a spaced apart and parallel leading surface 66E. The trailing surface 64E and the leading surface 66E are normal or perpendicular to the root 62E and the crest 60E.

Referring now to FIG. 17C, yet another embodiment of a profile of threads for a screw according to the present invention is shown. The profile 58F of threads 43F of the screw 30F has a generally truncated v-shape of a standard screw thread. The profile 58F includes a flat crest 60F and opposed angled trailing surfaces 64F and leading surface 66F. A root 62F extends from the trailing surface 64F and the leading surface 66F.

Referring now to FIG. 17D, yet another profile of threads of a screw is shown as profile 58G. The screw 30G includes threads 43G having the profile 58G. The profile 58G includes a leading surface 66G that is normal to a crest 60G and a spaced apart parallel root 62G. The profile 58G further includes a trailing surface 64G that is positioned at an angle between the roots 62G and the crest 60G.

According to the present invention and referring now to FIG. 17E, yet another form of profile of the screw of the present invention. The screw 30H of FIG. 17E include threads 43H defining profile 58H. The profile 58H includes a spaced apart parallel crest 60H and root 62H. The profile 58H include a trailing surface 64H, which is normal to the root 62H and the crest 60H. The profile 58H further includes a leading surface 66H, which is positioned at angle between root 62H and crest 60H.

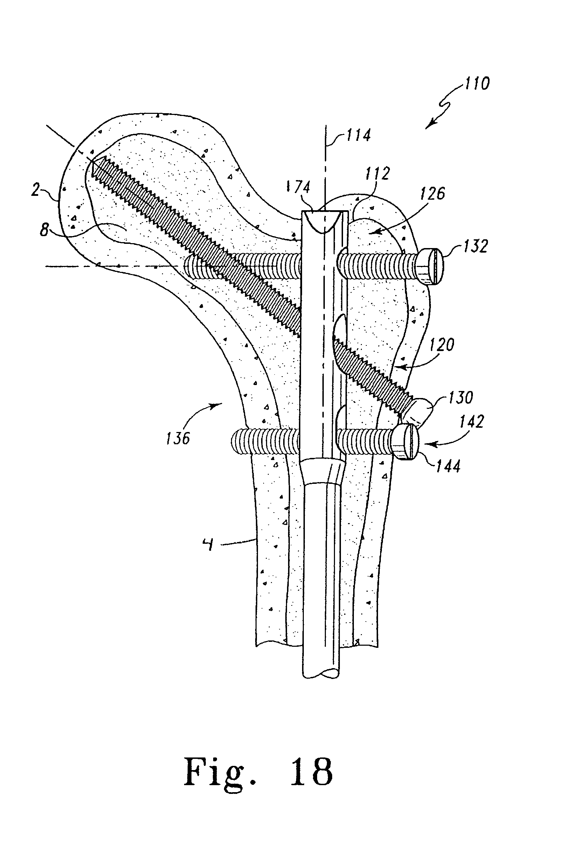

Referring now to FIG. 18, yet another embodiment of the present invention is shown as nail assembly 110. Nail assembly 110 of FIG. 18 is similar to the intramedullary nail assembly 10 of FIG. 1 except that the nail assembly 110 of FIG. 18 includes only three openings in proximal portion 136 of intramedullary nail 112. The proximal portion 136 includes first opening 120 as well as second opening 126 and third opening 142.

As shown in FIG. 18, the second opening 126 and third opening 142 are transverse. The second opening 126 has a generally circular cross section while the third opening 142 may have an oval shaped opening. The first opening 120 may be oblique or positioned at an angle such that it enters head 2 of the femur 4. It should be appreciated that the third opening 142 is in a plane different than that of openings 120 and 126.

Cortical screws may be placed in the second opening 126 and the third opening 142. For example as shown in FIG. 18 a second screw 132 is slidably fitted to second opening 126 and a third screw 144 is slidably fitted to third aperture 142. A first screw 130 is slidably fitted to first opening 120 and engages cancellous bone 8 in head 2 of the femur 4. The nail 112 may include a chamfer 174 to provide clearance for the patella tendon when the nail 112 is used in a retrograde manner.

It should be appreciated that the nail 112 may be used both on the left femur as shown in FIG. 18 as well as for the right femur. It should also be appreciated that the first opening 120 may be used for a greater trochanter to lesser trochanter securement of the nail 112 if the nail 112 is rotated approximately 180 degrees about intramedullary nail longitudinal axis 114.

In order that the nails for the femur are built to mate with the anatomy of a femur, it should be appreciated that right and left hand intramedullary nails may be desired. It should further be appreciated that the femoral intramedullary nail 112 of FIG. 18 while not well suited for a femoral nail for a right femur, the nail 112 may be suitable for use as a greater trochanter to lesser trochanter intramedullary nail for a left femur.

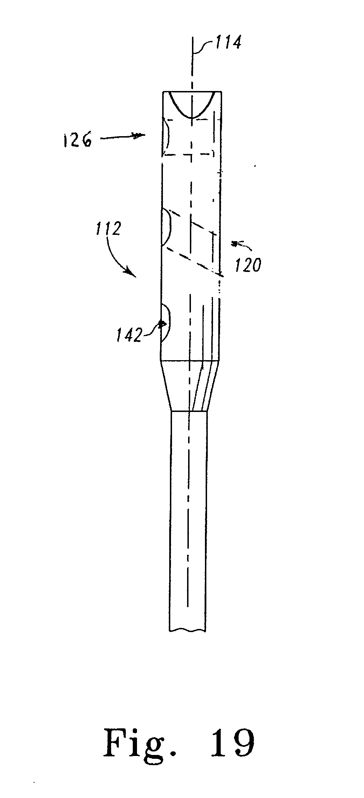

Referring now to FIG. 19, the first opening 120, second opening 126 and third opening 142 are shown in the proximal portion 136 of the intramedullary nail 112. Similarly as can be seen from FIG. 4A, the second opening 126 and the third opening 142 form a first plane while the first opening 120 forms a second plane. The first plane and the second plane, as is shown in FIG. 19, pass through longitudinal axis or centerline 114 of the nail 112.

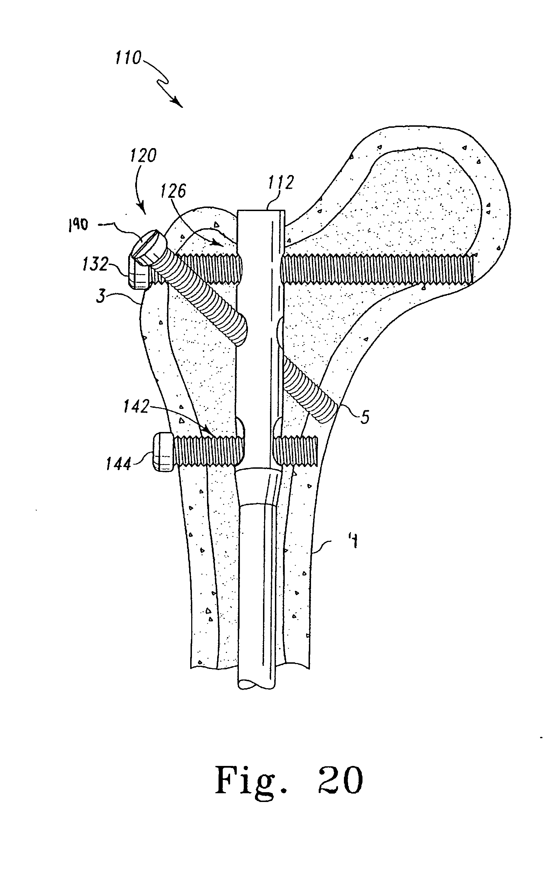

Referring now to FIG. 20, the nail assembly 110 is shown for use in the right femur 4. When used in the right femur 4, the intramedullary nail 112 is used as a greater trochanter to lesser trochanter nail. For example, as is shown in FIG. 20, the intramedullary nail assembly 110 includes intramedullary nail 112 having second opening 126 for receiving second screw 132 as well as third opening 142 for receiving third screw 144. The second screw 132 and the third screw 144 are transverse screws. The intramedullary nail 112 further includes the first opening 120 which receives fourth screw 190, which passes from greater trochanter 3 to lesser trochanter 5.

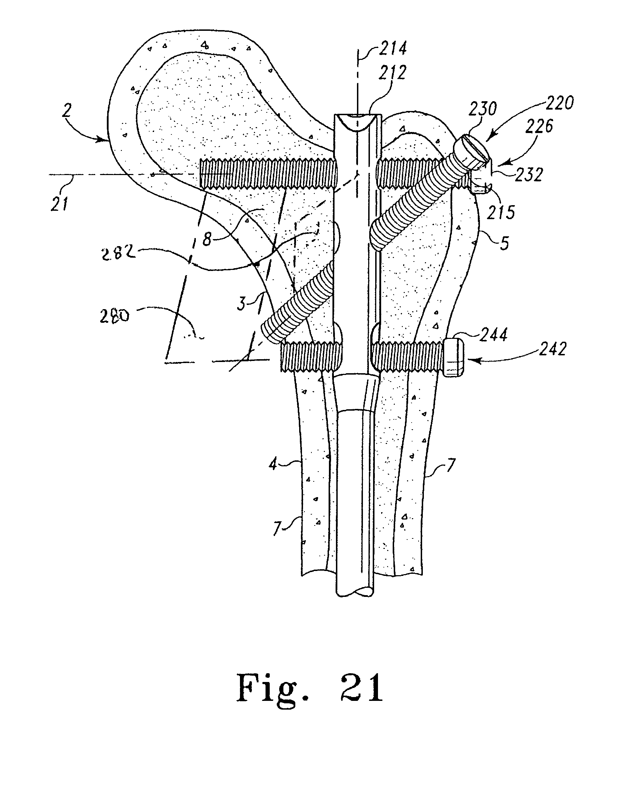

Referring now to FIGS. 21, 22 and 23, yet another embodiment of the present invention is shown as intramedullary nail assembly 210. The intramedullary nail assembly 210 is similar to the intramedullary nail 110 of FIGS. 18 through 20. Nail assembly 210, however, is designed for greater trochanter to lesser trochanter securement of the left femur and/or for neck securement of the right femur.

For example, and as shown in FIG. 21, the nail assembly 210 includes a nail 212, which is symmetrical to nail 112 of FIGS. 18 through 20. The nail assembly 210 of FIGS. 21 through 23 is adapted for greater trochanter to lesser trochanter securement of a left femur and neck securement of a right femur.

For example, and as shown in FIG. 21, the nail assembly 210 includes the nail 212, which has a second opening 226 for receiving second screw 232 as well as third opening 242 for receiving third screw 244. The second screw 232 and the third screw 244 are parallel and describe first plane 280. Nail 212 further includes first opening 220 for receiving first screw 230. The first opening 220 and longitudinal centerline 214 of the nail 212 define a second plane to 282. The first plane 280 and the second plane 282 intersect each other at the longitudinal centerline 214.

The second screw 232 extends from cortical wall 7 of femur 4 through cancellous bone 8, through second opening 226, through cancellous bone 8 and into cortical bone 7. The third screw 244 extends from cortical bone 7, through cancellous bone 8, through third opening 242, through cancellous bone 8, and into cortical bone 7. The first opening 220 extends from lesser trochanter 5 through cancellous bone 8 through first opening 220, through cancellous bone 8 and into the greater trochanter 3.

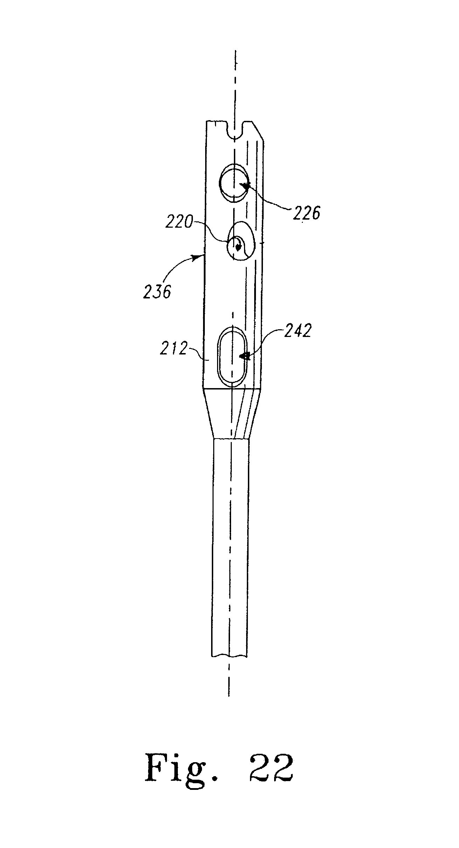

Referring now to FIG. 22, the proximal portion 236 of the nail 212 is shown in greater detail. The proximal portion 236 includes first opening 220, second opening 226 and third opening 242. The first opening 220 and the second opening 226 have a generally cylindrical cross section and the third opening 242 as shown in FIG. 22 is also cylindrical.

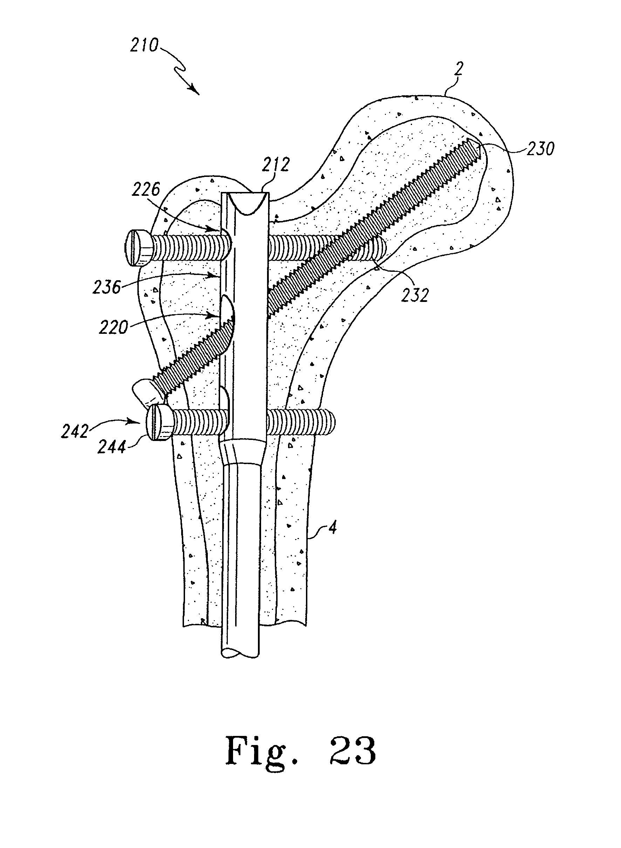

Referring now to FIG. 23, the nail assembly 210 is shown for use as a neck securing nail assembly for use with the right femur 4. The nail assembly 210 includes nail 212, which includes a proximal portion 236. The proximal portion 236 includes first opening 220, second opening 226 and third opening 242. The second screw 232 is slidably fitted into the second opening 226. Third screw 244 is slidably fit into third opening 242. First screw 230 is slidably fitted into first opening 220 and extends into head 2 of the femur 4.

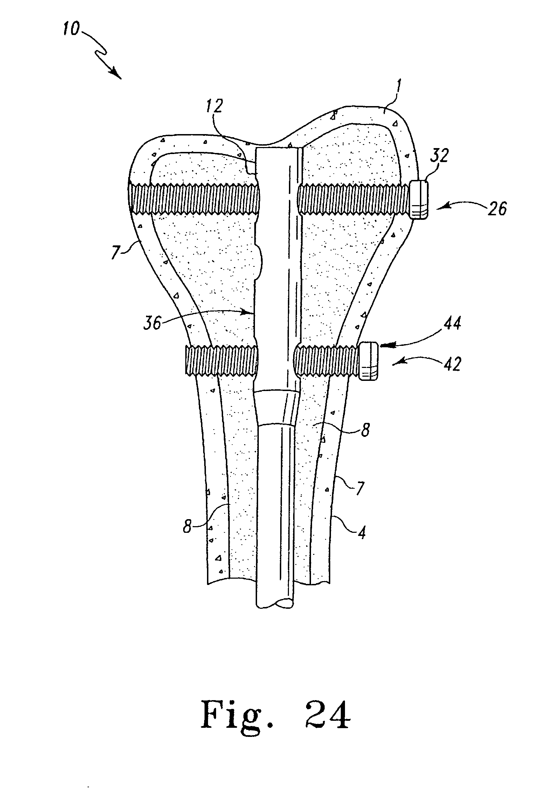

Referring now to FIG. 24, it should be appreciated that the nail assembly of the present invention may be used as a retrograde nail. For a retrograde femoral nail, the nail is inserted through the distal portion of the femur.

For example and as shown in FIG. 24, nail assembly 10 is shown assembled in the retrograde fashion on femur 4. The nail assembly 10 as shown in FIGS. 1 through 10, includes the nail 12, which is positioned in distal condylar portion 1 of the femur 4. The distal condylar portion 1 of the femur 4 receives the proximal portion 36 of the nail 12. The proximal portion 36 includes second opening 26 for receiving second screw 32. The proximal portion 36 of the nail 12 further includes third opening 42 for receiving third screw 44. The second screw 32 and the third screw 44 extend from one cortical wall 7 through the cancellous bone 8 and through the nail 12 to the opposite cancellous bone 8 and into the opposed cortical wall 7.

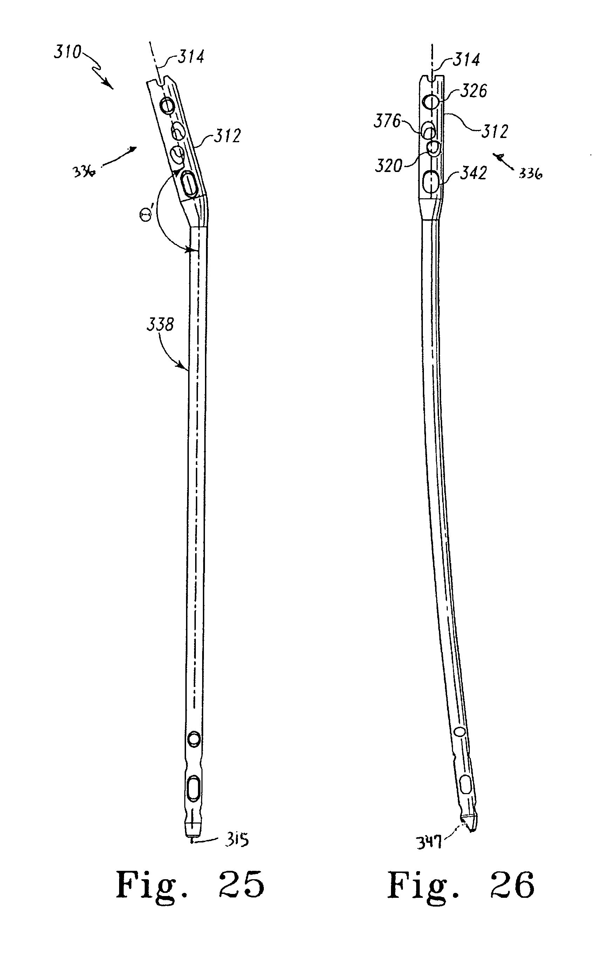

Referring now to FIGS. 25, 26 and 27, yet another embodiment of the present invention is shown as nail assembly 310. The nail assembly 310 is in the form of a trochanteric entry nail assembly. The trochanteric nail assembly 310 includes a nail 312 having a proximal portion 336 and a distal portion 338. Unlike the nail 12 of FIG. 1, nail 312 is bent between the proximal portion 336 and the distal portion 338. For example, as is shown in FIG. 25, the proximal portion 336 defines a proximal portion centerline 314 and the distal portion 338 defines a distal portion longitudinal centerline 315. The proximal portion centerline 314 and the distal portion centerline 315 define an angle there between.

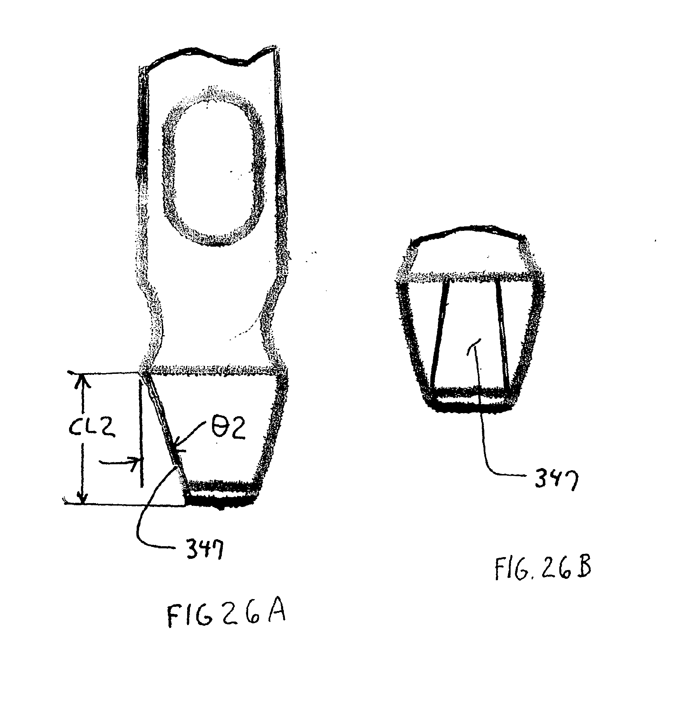

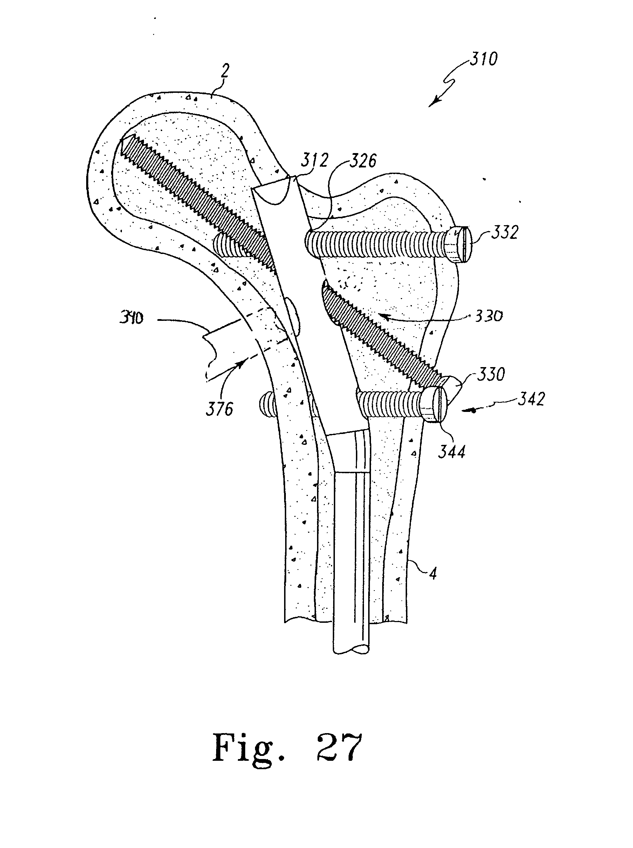

Referring now to FIGS. 26 and 27, the proximal portion 336 of the nail 312 includes a first opening 320 that extends obliquely through proximal centerline 314 and is used to receive neck or first screw 330. The proximal portion 336 further includes a second opening 326 and a third opening 342. The second and third openings 326 and 342 are for receiving transverse screws 332 and 344, respectively. The nail 312 further includes a fourth opening 376 for receiving the lesser trochanter to greater trochanter nail.

The nail 312 may, as shown in FIG. 26 include a relief surface such as a flat surface for example a chamfer 347 for assisting in leading the curved nail 312 into the medullary canal of the long bone, for example the femur. It should be appreciated that the chamfer may have a surface that is not flat, for example arcuate, for example a portion of a sphere or a cylinder.

Referring now to FIG. 26A the chamfer 347 is shown in the medial/lateral view with chamfer 347 shown on the side of the distal tip opposed to the origin of the curved portion of the nail 312. The chamfer may be defined by angle 2 from the longitudinal periphery of the nail 312. The chamfer may be further defined by chamfer length CL2 from the distal end of the nail 312.

Referring now to FIG. 26B the chamfer 347 is shown in the anterior/posterior view with chamfer 347 shown at distal tip. It should be appreciated that the tip may be larger or smaller than shown.

Referring now to FIG. 27, the intramedullary nail assembly 310 includes in addition to the intramedullary nail 312, the first screw 330 for cooperation with first opening 320. The first screw 330 extends into head 2 of the femur 4. The nail assembly 310 further includes the second screw 332 for cooperation with the second opening 326. The intramedullary nail 310 further includes the third screw 344 for slidably fitting in third aperture 342. The nail 312 further includes fourth opening 376. The fourth screw 390 can be used if the first screw 330 is removed.

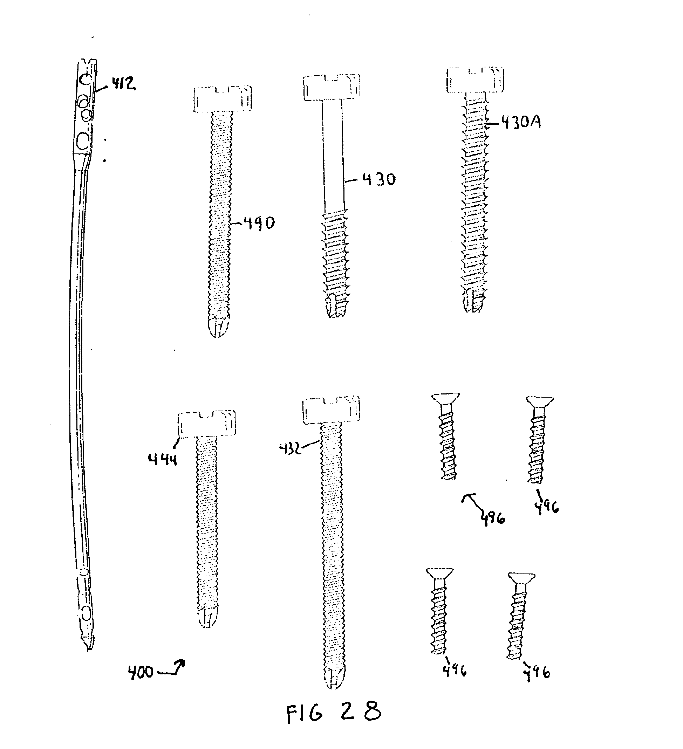

Referring now to FIG. 28, yet another embodiment of the present invention is shown as kit 400 for performing trauma surgery. The kit 400 includes a nail 412 similar to nail 12 of FIG. 1. The kit 400 further includes a first screw 430 similar to the screw 30 of FIG. 1.
The kit 400 further includes a second screw 432 similar to the screw 32 of FIG. 1. The kit 400 may also include additional screws. For example, the kit 400 may include a third screw 444 similar to the screw 44 of FIG. 1, as well as a fourth screw 490 similar to the screw 90 of FIG. 9. The kit 400 may further include distal screws. For example, the kit 400 may include distal screws 496 similar to the screw 96 of FIG. 12A. The kit 400 may include four separate distal screws 496. The kit 400 may further include a fully threaded cancellous screw 430A as an alternate to the partially threaded screw 430.

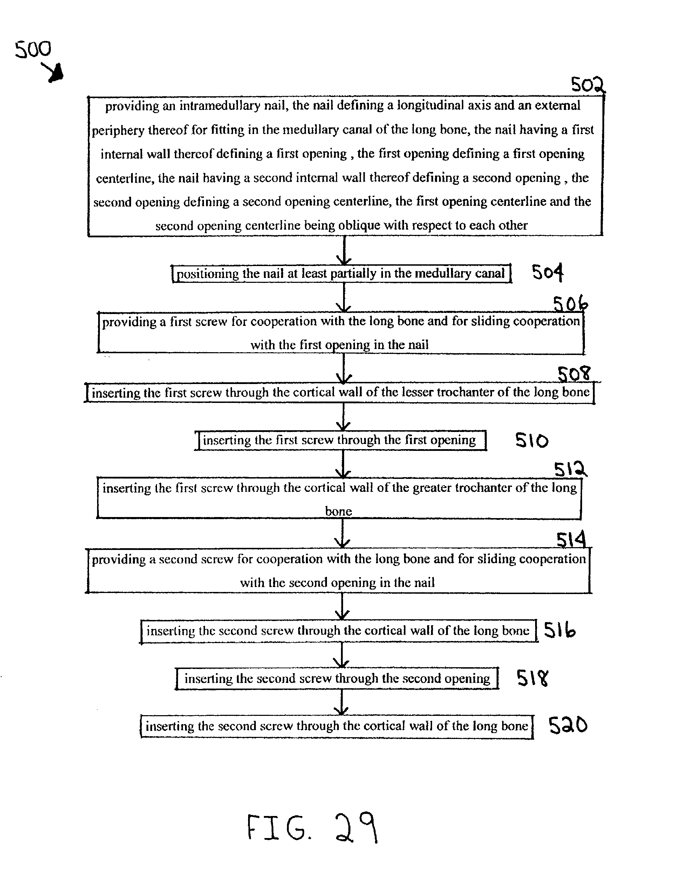

Referring now to FIG. 29, yet another embodiment of the present invention is shown as method 500 for performing trauma surgery. The method 500 may include a first step 502 of providing an intramedullary nail. The nail may define a longitudinal axis and an exterior periphery of the nail. The nail may be fitted into the medullary canal of the long nail, for example, a femur. The nail has a first internal wall of the nail defining a first opening through the nail. The first opening defines a first opening centerline. The nail has a second internal wall defining a second opening through the nail. The second opening defines a second opening centerline. The first opening centerline and the second opening centerline are oblique with respect to each other.

The method 500 further includes a second step 504 of positioning the nail at least partially in the canal. The method 500 further includes a third step 506 of providing a first screw for cooperation with the long bone or femur and for slidably cooperating with the first opening in the nail. The method 500 further includes a fourth step 508 of inserting the first screw through the cortical wall of the lesser trochanter of the long bone.

The method 500 further includes a fifth step 510 of inserting the first screw through the first opening. The method 500 further includes a sixth step 512 of inserting the first screw through the cortical wall of the greater trochanter of the long bone. The method further includes a seventh step 514 of providing a second screw for cooperation with the long bone and for slidable cooperation with the second opening in the nail. The method 500 further includes an eighth step 516 of inserting the second screw through the cortical wall of the long bone. The method further includes a ninth step 518 of inserting the second screw through the second opening. The method has a tenth step 520 of inserting the second screw through the cortical wall of the long bone.

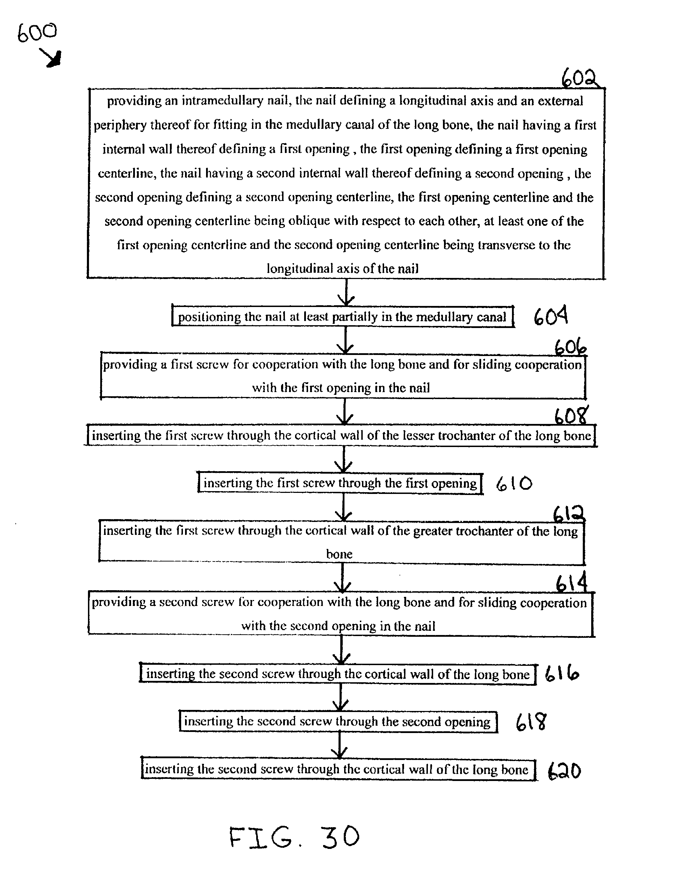

Referring now to FIG. 30, yet another embodiment of the present invention is shown as method 600 for performing trauma surgery. The method 600 may include a first step 602 of providing an intramedullary nail. The nail defines a longitudinal axis and an external periphery for fitting in the medullary canal of the long bone. The nail has a first internal wall defining a first opening. The first opening defines a first opening centerline. The nail has a second internal wall defining a second opening. The second opening has a second opening centerline. The first opening centerline and the second opening centerline are oblique with respect to each other. At least one of the first opening centerline and the second opening centerline are transverse to the longitudinal axis of the nail. The method 600 may include a second step 604 of positioning the nail at least partially in the medullary canal and a third step 606 of providing a first screw for cooperation with the long bone and for sliding cooperation with the first opening in the nail. The method 600 may include a fourth step 608 of inserting the first screw through the cortical wall of the lesser trochanter of the long bone and a fifth step 610 of inserting the first screw through the first opening. The method may also include a sixth step 612 of inserting the first screw through the cortical wall of the greater trochanter of the long bone and a seventh step 614 of providing a second screw for cooperation with the long bone and for sliding cooperation with the second opening in the nail. The method may also include an eighth step 616 of inserting the second screw through the cortical wall of the long bone and a ninth step 618 of inserting the second screw through the second opening. The method may also include a tenth step 620 of inserting the second screw through the cortical wall of the long bone.

## Claims

1. An intramedullary nail for use in a medullary canal of a long bone, said nail comprising a body defining a longitudinal axis and an external periphery thereof for fitting in the medullary canal of the long bone, said body having a first internal wall thereof defining a first opening there through, the first opening defining a first opening centerline, said body having a second internal wall thereof defining a second opening there through, the second opening defining a second opening centerline, the first opening centerline and the second opening centerline being oblique with respect to each other, at least one of the first opening centerline and the second opening centerline being transverse to the longitudinal axis of said body.

2. The nail as in claim 1, wherein at least a portion of said body is cannulated along the longitudinal axis.

3. The nail as in claim 1, wherein at least a portion of said body defines a groove along the longitudinal axis.

4. The nail as in claim 1, wherein at least one of the first opening and the second opening have a generally cylindrical shape.

5. The nail as in claim 1, wherein at least one of the first opening and the second opening have a generally oval shape.

6. The nail as in claim 1, wherein at least one of the first opening centerline and the second opening centerline intersects the longitudinal axis of said body.

7. The nail as in claim 1, wherein said body further comprises a internal wall defining a third opening the first opening and the second opening have a generally cylindrical shape.

8. The nail as in claim 1, wherein the external periphery of said body is substantially cylindrical.

9. The nail as in claim 8, wherein said nail defines a first portion having a first diameter and a second portion having a second diameter, the first diameter being larger than the second diameter.

10. The nail as in claim 1, wherein the first opening and the second opening are located in the first portion.

11. The nail as in claim 10, wherein the other of the at least one of the first opening centerline and the second opening centerline forms an acute angle with the longitudinal axis of said body.

12. A kit for use in repairing a fracture in a long bone, which comprises a nail as claimed in claim 1, a first screw adapted to be slidably fittable with the first opening; and a second screw adapted to be slidably fittable with the second opening.
